# EUROPÄISCHE PATENTANMELDUNG

(11) **EP 4 755 430 A1**
(43) Veröffentlichungstag der Anmeldung: **10.06.2026**
(21) Anmeldenummer: 25220350.0
(22) Anmeldetag: 03.12.2025
(51) Int. Cl.: A61M 16/06

(54) **HALTERUNGSSYSTEM FÜR EIN PRONG-SYSTEM ZUR BEATMUNGSUNTERSTÜTZUNG**

(30) Priorität: 05.12.2024 DE 102024136320; 01.04.2025 DE 102025112627
(71) Anmelder: Fritz Stephan GmbH, 56412 Gackenbach (DE)
(72) Erfinder: Tekinder, Christian, 56412 Gackenbach (DE)
(74) Vertreter: Werner, André

(57) **Zusammenfassung**

Die Erfindung betrifft ein Halterungssystem für ein Prong-System zur Beatmungsunterstützung eines Patienten (4), insbesondere zur Beatmungsunterstützung im Rahmen der Hochfluss-Sauerstofftherapie. Das Halterungssystem zur Halterung eines Prongs (1) des Prong-Systems umfasst Fixierpads (2), die über eine lösbare Klemmverbindung mit Klemmhaltern (1.3) verbindbar sind, wobei jeder der Klemmhalter (1.3) mindestens einen Rasthaken (1.3.1) aufweist, der in einen geometrisch korrespondierenden Hinterschnitt (2.1) am jeweiligen Fixierpad (2) ein- und ausrastbar ist und über einen elastisch verformbaren Dehnbereich (1.3.2) zum Ein- und Ausrasten der Klemmverbindung verfügt. Das Halterungssystem ermöglicht eine einfache, sichere und patientenschonende Fixierung des Prongs (1); es eignet sich insbesondere für die Beatmungsunterstützung von Frühgeborenen, Neugeborenen, Säuglingen und Kindern.

## Beschreibung

Die vorliegende Erfindung betrifft ein Halterungssystem für ein Prong-System zur Beatmungsunterstützung. Das Prong-System eignet sich für die Beatmungsunterstützung von Patienten im Rahmen der Hochfluss-Sauerstofftherapie bzw. High-Flow-Therapie und ist insbesondere auf die Bedürfnisse von Frühgeborenen, Neugeborenen, Säuglingen und Kindern abgestimmt.

Prong-Systeme für die Hochfluss-Sauerstofftherapie umfassen im Regelfall den eigentlichen Prong, ein Halterungssystem, um den Prong am Patienten zu befestigen, und einen mit dem Beatmungsgerät verbundenen Verbindungsschlauch, über den das Atemgas zugeführt wird. Der Prong selbst besitzt Nasenkanülen, die während der Beatmungsunterstützung in den Nasenlöchern des Patienten platziert werden, und ein Verteilerstück, an dem die Nasenkanülen ansetzen. Das Atemgas wird in das Verteilerstück des Prongs aus dem Verbindungsschlauch eingeleitet, durch das Verteilerstück auf die üblicherweise zwei Nasenkanülen verteilt und aus den Nasenkanülen zur Beatmungsunterstützung ausgeleitet.

Derzeitige Systeme zur Befestigung bzw. zur Halterung von Prong-Systemen am Patienten verwenden häufig Klebeverbindungen auf den Wangen der Patienten. Diese Klebeverbindungen, obwohl effektiv, können bei wiederholtem Lösen und Anbringen die Haut und das Gewebe der Patienten erheblich belasten und zu Verletzungen führen.

Solche auf die Wangen geklebten Fixierpads können zur Halterung des Prongs auch mittels Klettverbindungen am Prong befestigt werden. Bei dieser Art der Befestigung kommt aufgrund der anfangs hohen Haltekraft der Klettverbindung jedoch mitunter zum ungewollten Lösen des Fixierpads von der Haut des Patienten. Dies verursacht nicht nur Stress für den Patienten, sondern auch erhöhten Zeitaufwand für das medizinische Personal. WO 2012/053910 A1 beschreibt ein solches System zur Befestigung von Prongs, bei denen auf die Wangen aufgeklebte Fixierpads über ein zweiteiliges Befestigungselement am Träger der Nasenkanülen mit Klettverbindungen befestigt werden. Ähnliche Halterungssysteme mit auf die Wangen aufgeklebten Fixierpads offenbaren WO 2014/142681 A1 und WO 2015/152734 A1.

Die Aufgabe der vorliegenden Erfindung besteht darin, ein Halterungssystem für ein Prong-System zur Hochfluss-Sauerstofftherapie bereitzustellen, das die Nachteile herkömmlicher Befestigungsmethoden überwindet. Insbesondere soll das System die Belastung der empfindlichen Haut von Frühgeborenen, Neugeborenen, Säuglingen und Kindern minimieren, die durch wiederholtes Lösen und Anbringen von Klebe- oder Klettverbindungen entsteht. Zudem soll die Erfindung eine einfache, schnelle und reproduzierbare Positionierung der Nasenkanülen gewährleisten, um eine korrekte und sichere Beatmungsunterstützung zu ermöglichen. Dadurch sollen sowohl die Belastung für den Patienten als auch der zeitliche Aufwand für das medizinische Personal reduziert werden.

Diese Aufgabe wird durch ein Halterungssystem für ein Prong-System mit den Merkmalen nach Anspruch 1 sowie ein Prong-System nach Anspruch 9 gelöst. Zweckmäßige Weiterbildungen der Erfindung sind in den Ansprüchen 2 bis 8 sowie 10 aufgeführt.

Das erfindungsgemäße Halterungssystem ist bestimmungsgemäß zur Halterung eines Prong-Systems zur Beatmungsunterstützung eines Patienten bzw. zur Atemunterstützung vorgesehen. Insbesondere ist es für die Beatmungsunterstützung im Rahmen der Hochfluss-Sauerstofftherapie bzw. High-Flow-Therapie bestimmt.

Das mittels des vorgeschlagenen Halterungssystems zu halternde Prong-System umfasst einen Prong, der eine oder mehrere (vorzugsweise zwei) Nasenkanülen zur Abgabe eines sauerstoffhaltigen Atemgases an den Patienten sowie ein an die Nasenkanülen angeschlossenes Verteilerstück zur Zuleitung des Atemgases zu den Nasenkanülen und zur Verteilung des Atemgases auf die Nasenkanülen aufweist.

Das Halterungssystem umfasst ein oder mehrere auf die Wangen des Patienten aufbringbare Fixierpads. Diese in bekannter Weise auf die Wangen der Patienten aufzuklebenden Fixierpads sind flächige Gebilde, die jeweils eine Klebefläche zur Befestigung der Fixierpads auf der Haut des Patienten aufweisen. Zudem kann das Prong-System einen Verbindungsschlauch zur Zuleitung des Atemgases zum Prong beinhalten. Das Verteilerstück, an dem die Nasenkanülen abzweigen, dient als Verteiler des über den Verbindungsschlauch zugeführten Atemgases zu den Nasenkanülen.

Bei dem die klebefähigen Fixierpads umfassenden Halterungssystem sowie dem damit ausgestatteten Prong-System, das insbesondere für die Hochfluss-Sauerstofftherapie bei Frühgeborenen, Neugeborenen, Säuglingen und Kindern zur Anwendung kommen soll, sind die Verbindungen zwischen dem Prong und den Fixierpads durch Klemmverbindungen realisiert. Erfindungsgemäß umfasst das Halterungssystem zur Ausbildung der Klemmverbindungen neben den Fixierpads ein oder mehrere Klemmhalter, die fest mit dem Prong verbunden sind oder einen integralen Bestandteil des Prongs bilden.

Jeder der Klemmhalter ist dabei einem der Fixierpads zugeordnet und über eine lösbare Klemmverbindung mit dem entsprechenden Fixierpad verbindbar. Der Klemmhalter besitzt hierfür mindestens einen Rasthaken, der dazu ausgebildet ist, in einen an dem zugeordneten Fixierpad ausgeformten Hinterschnitt einzurasten. Dieser Hinterschnitt ist geometrisch so gestaltet, dass er mit dem Klemmhalter korrespondiert. Der jeweilige Rasthaken ist zur Ausbildung der lösbaren Klemmverbindung in den geometrisch mit dem Klemmhalter korrespondierenden Hinterschnitt des zugeordneten Fixierpads normalkraftfrei, zum Beispiel parallel oder unter einem flachen Winkel, zur Klebefläche des Fixierpads einrastbar. Realisiert werden die wiederholt lös- und fixierbaren Klemmverbindungen mithin durch die an den Klemmhaltern ausgebildeten Rasthaken und die korrespondierenden, an den Fixierpads ausgeformten Hinterschnitte. Üblicherweise umfasst das Halterungssystem zwei der Klemmhalter und zwei der Fixierpads, wobei eine Klemmhalter-Fixierpad-Kombination zur Befestigung auf der linken Wange des Patienten und die andere Klemmhalter-Fixierpad-Kombination zur Befestigung auf der rechten Wange des Patienten vorgesehen ist.

Des Weiteren weist der Klemmhalter einen Dehnbereich aus einem reversibel und leicht dehnbaren elastischen Material auf, der so ausgebildet ist, dass er durch manuell am Klemmhalter ausgeübten mechanischen Druck und/oder Zug elastisch verformbar ist. Die elastische Verformbarkeit des Dehnbereichs ermöglicht bei dessen elastischer Verformung ein Ein- oder Ausrasten der Klemmverbindung, ohne dass Normalkräfte auf die Klebefläche des Fixierpads einwirken. Durch Dehnung des elastischen Dehnbereichs kann der Rasthaken des Klemmhalters aus dem am zugehörigen Fixierpad ausgebildeten Hinterschnitt herausgeführt und so vom Fixierpad gelöst werden. Der Teil des Klemmhalters, der den Dehnbereich des Klemmhalters bildet, ist weich- bis mittelelastisch ausgeführt. Unter weich- bis mittelelastisch wird hierbei ein Material- oder Strukturverhalten verstanden, bei dem eine Dehnung unter geringer, manueller Krafteinwirkung möglich ist. Der zur elastischen Verformung am Klemmhalter manuell ausgeübte mechanischen Druck und/oder Zug liegt vorzugsweise unterhalb von 150 N. Zur Einstellung des weich- bis mittelelastischen Material- oder Strukturverhaltens kann der Dehnbereich aus einem weich- bis mittelelastischen Werkstoff, zum Beispiel einem elastomeren Material bzw. einem Elastomer, gefertigt sein. Geeignete elastomere Materialien sind u. a. Silikonkautschuk oder thermoplastische Elastomere (TPE), insbesondere Polyurethanelastomere (TPE-U bzw. TPU). Das weich- bis mittelelastische Material- oder Strukturverhalten kann ferner durch federelastische Ausführungen mit geringer Federkonstante, zum Beispiel Spiralfedern aus Kunststoffen oder Metallen, realisiert werden.

Durch die elastische Rasthaken-Hinterschnitt-Verbindung ist eine form- und kraftschlüssige Klemmverbindung geschaffen, die ein mehrfaches Lösen und Fixieren des Prongs ermöglicht, ohne dabei den Patienten zu strapazieren. Der Dehnbereich ist hierbei so ausgerichtet, dass die Dehnung im Wesentlichen in einer Richtung erfolgt, die vorzugsweise parallel oder nahezu parallel zur Klebefläche des Fixierpads liegt. Hierdurch wird vermieden, dass bei Dehnung des Dehnbereichs des Klemmhalters eine Zug- oder Druckbelastung auf die Klebefläche bzw. auf die darunterliegende Patientenhaut ausgeübt wird.

Das Halterungssystem erlaubt es, die Verbindung zwischen Prong und Fixierpad durch einfaches Zusammendrücken oder Dehnen des Klemmhalters zu lösen, ohne dass dabei Belastungen auf die Haut des Patienten entstehen. Die Fixierpads werden einmalig in optimaler Position auf die Wangen des Patienten angebracht und verbleiben dort während der gesamten Therapie. Durch die vorgegebenen Positionen der an den Fixierpads ausgeformten Hinterschnitte wird - im Unterschied zu Klettverbindungen - sichergestellt, dass das Prong-System bei jedem Anbringen automatisch korrekt und reproduzierbar positioniert wird.

Das Material der Klemmhalter ist so gestaltet, dass es die Spannung, die beim Lösen und Wiederanbringen entsteht, flexibel aufnimmt und abbaut, wodurch Hautirritationen und mechanische Belastungen vermieden werden. Das Wiederanbringen des Prongs erfolgt schnell und präzise, ohne dass Korrekturen der Nasenkanülenposition erforderlich sind.

Das erfindungsgemäße Halterungssystem bietet Vorteile gegenüber bisherigen Lösungen: Es reduziert die Belastung der empfindlichen Haut, ermöglicht eine einfache und zeitsparende Handhabung und sorgt für eine konsistente und sichere Platzierung der Nasenkanülen. Insgesamt trägt die Erfindung zu einer verbesserten Patientenversorgung und einem effizienteren Arbeitsablauf in der Hochfluss-Sauerstofftherapie bei.

Die Rasthaken besitzen - entsprechend dem üblichen Aufbau eines Hakens - jeweils eine Hakenspitze, einen Bogenbereich und einen Schenkelbereich. Vorzugsweise weist jeder der Klemmhalter zwei Rasthaken auf. Diese beiden Rasthaken sind so gestaltet, dass ihre Hakenspitzen spiegelsymmetrisch einander zugewandt sind. D. h., die Rasthaken sind so angeordnet, dass deren Hakenspitzen in entgegengesetzte Richtungen zeigen und in einer spiegelbildlichen Ausrichtung zueinander stehen. Durch die spiegelsymmetrische Anordnung der Rasthaken wird gewährleistet, dass bei der Betätigung der Klemmverbindung eine gleichmäßige mechanische Beanspruchung erfolgt.

Gemäß einer Ausgestaltung des Halterungssystems, dessen Klemmhalter jeweils die zwei spiegelsymmetrischen Rasthaken aufweisen, besitzt jeder der Rasthaken einen biegeelastischen Schenkel- und Bogenbereich, an dessen Innenkontur sich der Dehnbereich erstreckt. Der biegeelastische Schenkel- und Bogenbereich des Rasthakens ist dabei so ausgebildet, dass er durch manuell am Klemmhalter ausgeübten mechanischen Druck und/oder Zug elastisch biegbar bzw. verformbar ist. Diese elastische Biege- bzw. Verformbarkeit ermöglicht ein bogenförmiges Ein- oder Ausrasten des Rasthakens in den oder aus dem ihm zugeordneten Hinterschnitt. Diese Ausbildung des Klemmhalters ermöglicht die einhändige Betätigung zum Ein- und Ausrasten der Klemmverbindung.

Insbesondere kann im Rahmen dieser Ausgestaltung des Halterungssystems jeder der Klemmhalter in spiegelsymmetrischer Form, zum Beispiel in Herzform, in Kreisform oder in Form eines regelmäßigen Polygons, insbesondere in Form eines gleichschenkligen Dreiecks, ausgebildet sein. Dabei sind die zwei Rasthaken des Klemmhalters spiegelsymmetrisch zur Spiegelsymmetrielinie der jeweiligen Form angeordnet. Im Fall der Herzform und der Form des gleichschenkligen Dreiecks ist dies zum Beispiel die von der Herzspitze oder dem Scheitelpunkt des gleichschenkligen Dreiecks ausgehende Symmetrielinie, d. h. die Symmetrieachse der Herzform oder die Winkelhalbierende des Winkels am Scheitelpunkt des gleichschenkligen Dreiecks. Durch die spezifische Form des Klemmhalters in spiegelsymmetrischer Form wird eine ergonomische Gestaltung erreicht, die eine einfache, insbesondere einhändige Handhabung unterstützt.

Gemäß einer weiteren Ausgestaltung des Halterungssystems, dessen Klemmhalter jeweils die zwei spiegelsymmetrischen Rasthaken aufweisen, besitzt jeder der Klemmhalter einen den Dehnbereich bildenden Rohrabschnitt. Dieser Rohrabschnitt ist an das Verteilerstück des Prongs zur Zuleitung des Atemgases angeschlossen. An beiden axialen Endbereichen des den Dehnbereich bildenden Rohrabschnitts ist jeweils einer der Rasthaken ausgebildet. Diese beiden Rasthaken sind spiegelsymmetrisch bezüglich des Rohrabschnitts angeordnet, sodass die Hakenspitzen dieser beiden Rasthaken einander zugewandt sind. Das Ein- und Ausrasten kann durch zweihändiges Dehnen des Rohrabschnitts realisiert werden; als Griffbereiche dienen hierbei die Endbereiche des Rohrabschnitts. Da das Dehnelement einen Teil der das Atemgas zuführenden Leitung darstellt, kann der Klemmhalter platzsparend in den Prong integriert werden.

Das erfindungsgemäße Prong-System umfasst neben dem beschriebenen Halterungssystem den Prong sowie den Verbindungsschlauch. Hierbei kann vorgesehen sein, dass der oder die Klemmhalter einen Bestandteil des Prongs bilden. D. h., der Prong, der die Nasenkanülen, das Verteilerstück und den Klemmhalter umfasst, ist zum Beispiel als monolithisches Bauteil ausgebildet. Die einstückige Ausführung des Prongs zeichnet sich durch besonders hohe Robustheit und Gasdichtigkeit aus.

Die Erfindung wird nachfolgend anhand von Ausführungsbeispielen und mit Bezug auf die schematischen Zeichnungen näher erläutert, wobei gleiche oder ähnliche Merkmale mit gleichen Bezugszeichen versehen sind. Diese zeigen:
- Fig. 1:: das Prong-System mit einem Halterungssystem gemäß einer ersten Ausführungsform in Beatmungsposition in perspektivischer Ansicht;
- Fig. 2:: das Prong-System mit dem Halterungssystem gemäß der ersten Ausführungsform in perspektivischer Ansicht;
- Fig. 3:: Details des Prong-Systems mit dem Halterungssystem gemäß der ersten Ausführungsform in perspektivischer Ansicht und partieller Schnittansicht;
- Fig. 4:: Details des Prong-Systems mit dem Halterungssystem gemäß der ersten Ausführungsform in perspektivischer Ansicht;
- Fig. 5:: das Prong-System mit einem Halterungssystem gemäß einer zweiten Ausführungsform in Beatmungsposition in perspektivischer Ansicht;
- Fig. 6:: das Prong-System mit dem Halterungssystem gemäß der zweiten Ausführungsform in perspektivischer Ansicht;
- Fig. 7:: das Halterungssystem gemäß der zweiten Ausführungsform in perspektivischer Ansicht;
- Fig. 8:: Details des Klemmhalters des Halterungssystems gemäß der zweiten Ausführungsform in perspektivischer Ansicht;
- Fig. 9:: Details des Fixierpads des Halterungssystems gemäß der zweiten Ausführungsform in perspektivischer Ansicht;
- Fig. 10:: die Funktion des Klemmhalters des Halterungssystems gemäß der zweiten Ausführungsform in perspektivischer Ansicht;
- Fig. 11:: die Funktion des Klemmhalters in Verbindung mit dem Fixierpad gemäß dem Halterungssystem der zweiten Ausführungsform in perspektivischer Ansicht;
- Fig. 12:: die Funktion des Klemmhalters in Verbindung mit dem Fixierpad gemäß dem Halterungssystem der zweiten Ausführungsform in perspektivischer Ansicht;
- Fig. 13:: die Verbindung von Klemmhalter und Fixierpad gemäß dem Halterungssystem der zweiten Ausführungsform in Längsschnittdarstellung.
- Fig. 14:: das Halterungssystem gemäß der zweiten Ausführungsform mit Spiralfeder-Dehnbereich in perspektivischer Ansicht;
- Fig. 15:: das Halterungssystem gemäß der zweiten Ausführungsform mit Spiralfeder-Dehnbereich in perspektivischer Ansicht.

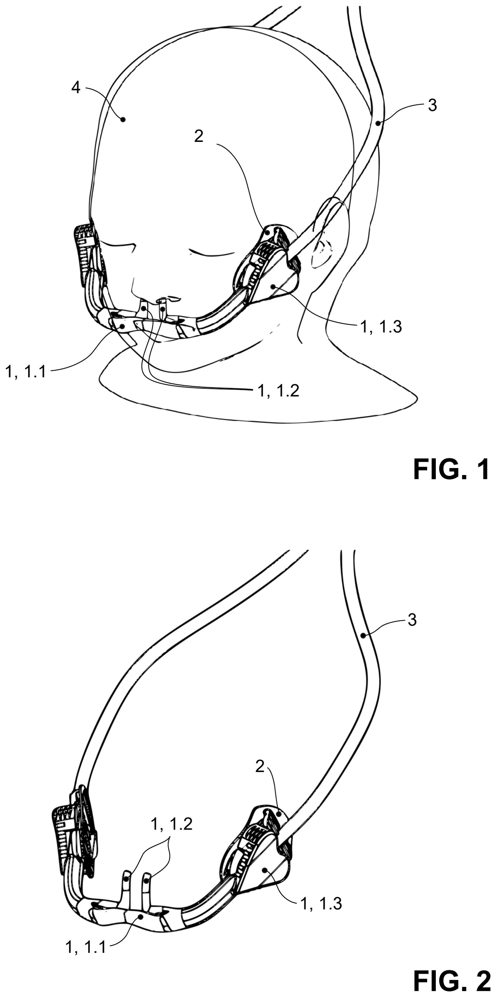

Die Fig. 1 bis 4 veranschaulichen eine erste Ausführungsform des Halterungssystems bzw. des Prong-Systems mit einem Klemmhalter in Herzform. Zur Beatmungsunterstützung umfasst das Prong-System - siehe Fig. 1 und Fig. 2 - als zentralen Bestandteil den Prong 1, der die zwei an das Verteilerstück 1.1 angeschlossenen Nasenkanülen 1.2 aufweist. Das Verteilerstück 1.1 dient der Zuleitung und Verteilung des Atemgases, das dem Prong 1 über den Verbindungsschlauch 3 zugeführt wird, zu den Nasenkanülen 1.2. Die Nasenkanülen 1.2 sind so ausgebildet, dass sie in die Nasenlöcher des Patienten 4 eingeführt werden, um das sauerstoffhaltige Atemgas dem Patienten 4 über die Nase zuzuführen.

Das Halterungssystem umfasst weiterhin zwei der Fixierpads 2, die jeweils über eine Klebefläche verfügen, mit der sie auf der Haut des Patienten 4 appliziert werden können. Jedes der Fixierpads 2 ist mit dem ihm zugeordneten Klemmhalter 1.3 verbunden bzw. verbindbar.

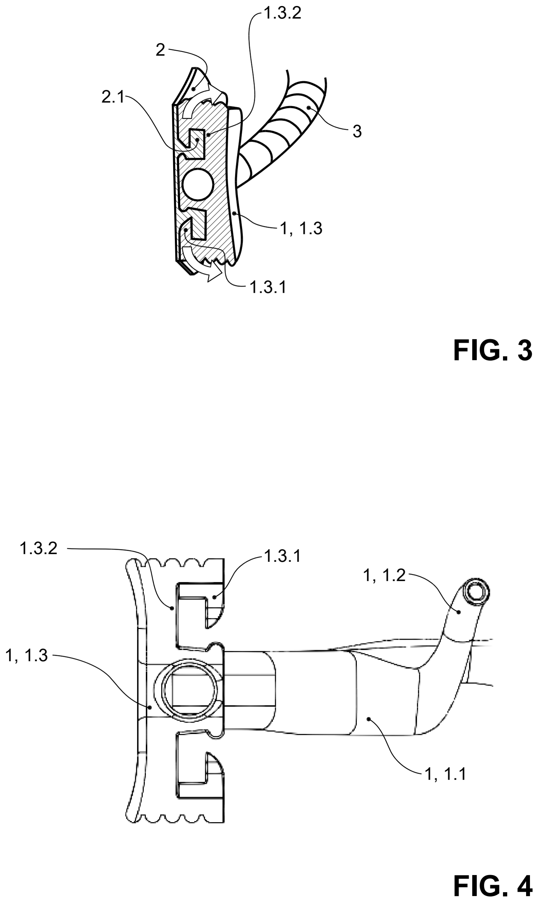

Die gemäß der ersten Ausführungsform des Halterungssystems einen Teil des Prongs 1 bildenden Klemmhalter 1.3 umfassen jeweils zwei der Rasthaken 1.3.1, die dazu dienen, in die am zugeordneten Fixierpad 2 ausgebildeten Hinterschnitte 2.1 einzurasten. Diese Hinterschnitte 2.1 sind so geformt, dass sie eine sichere und stabile Verbindung mit den Rasthaken 1.3.1 gewährleisten. In eingerasteter Position - siehe insbesondere Fig. 3 - greifen die Rasthaken 1.3.1 vollständig in die geometrisch korrespondierenden Hinterschnitte 2.1 ein.

Zum Ausrasten kann der herzförmige, aus Silikonkautschuk gebildete Klemmhalter 1.3 mit zwei Fingern so zusammengedrückt werden, dass die Rasthaken 1.3.1 nach außen aus den Hinterschnitten 2.1 des Fixierpads 2 (bogenförmig) herauswandern (siehe Pfeildarstellung in Fig. 3) und sich die Verbindung löst. Der jeweilige Rasthaken 1.3.1 besitzt hierzu einen biegeelastischen Schenkel- und Bogenbereich, an dessen Innenkontur sich der Dehnbereich 1.3.2 des Klemmhalters 1.3 befindet. Der Klemmhalter 1.3 besitzt - siehe Fig. 4 - zwei symmetrische Rasthaken 1.3.1, deren Hakenspitzen aufeinander zuweisen.

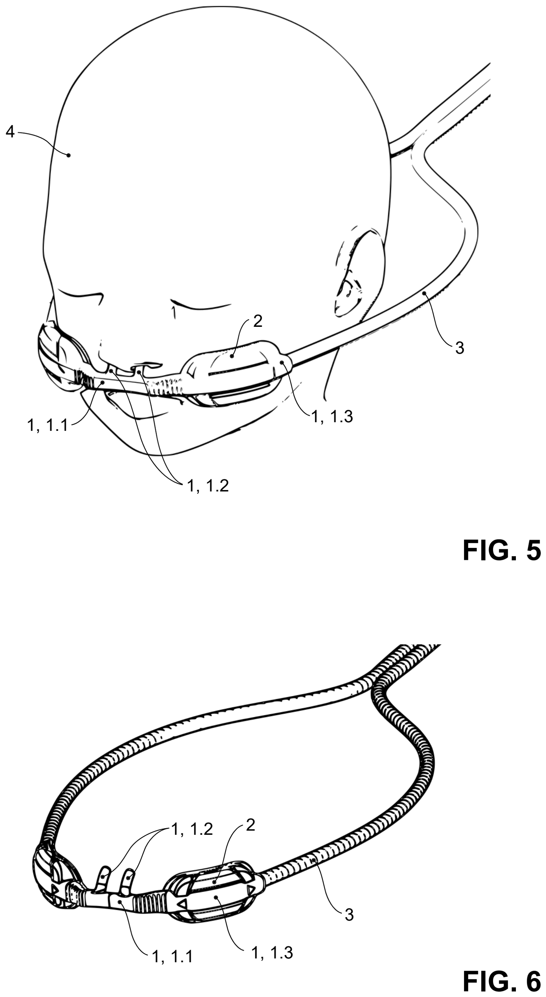

Die in den Fig. 5 bis 13 dargestellte zweite Ausführungsform des Halterungssystems bzw. des Prong-Systems besitzt Klemmhalter 1.3 mit ankerähnlichen bzw. ankerförmigen Rasthaken 1.3.1 und einem den Dehnbereich 1.3.2 bildenden Rohrabschnitt aus Silikonkautschuk. Der Prong 1 - siehe Fig. 5 und Fig. 6 - umfasst wiederum das Verteilerstück 1.1 mit den daran angeschlossenen Nasenkanülen 1.2. Über den Verbindungsschlauch 3 wird das Atemgas zugeführt, das zur Beatmungsunterstützung des Patienten 4 aus den Nasenkanülen 1.2 ausströmt.

Zur Halterung des Prongs 1 werden die Fixierpads 2 auf die Wangen des Patienten 4 aufgeklebt. Das Verteilerstück 1.1, die Nasenkanülen 1.2 und der Klemmhalter 1.3 sind gemäß der zweiten Ausführungsform integrale Bestandteile des Prongs 1.

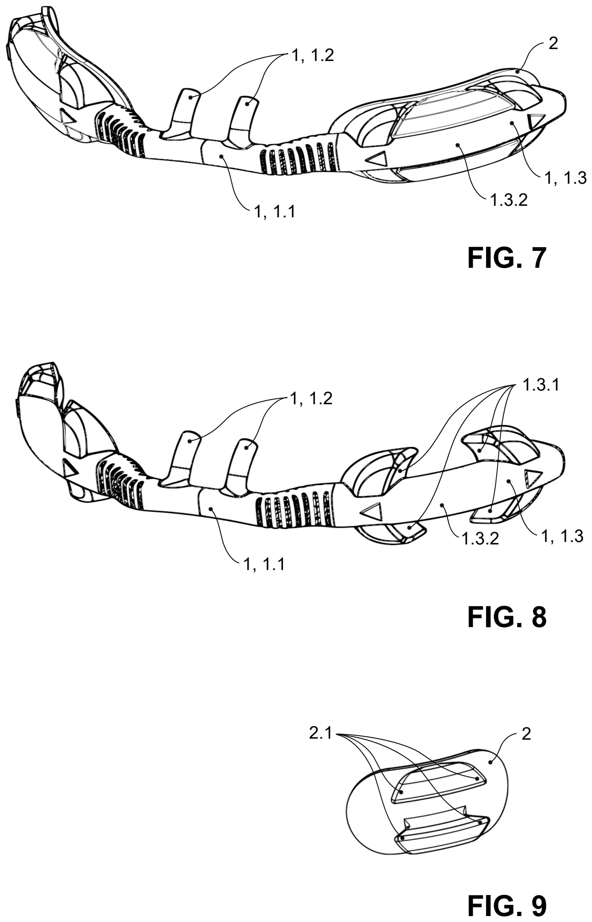

Die Fig. 7 bis 9 veranschaulichen die konstruktive Ausführung des Halterungssystems gemäß der zweiten Ausführungsform: Fig. 7 zeigt die an den Fixierpads 2 geklemmten Klemmhalter 1.3. Fig. 8 verdeutlicht die Ausformung der ankerförmigen Rasthaken 1.3.1, die symmetrisch an den beiden Endbereichen des dazwischenliegenden, den Dehnbereich 1.3.2 bildenden Rohrabschnitts angeordnet sind. Die Ausbildung der geometrisch mit den Rasthaken 1.3.1 korrespondierenden Hinterschnitte 2.1 am Fixierpad 2 geht insbesondere aus Fig. 9 hervor.

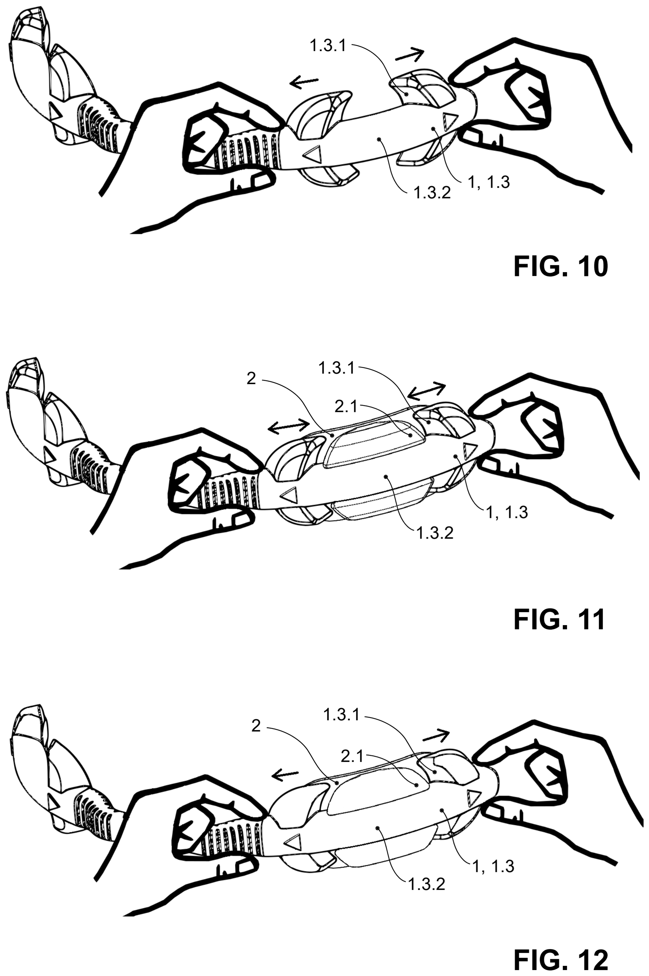

Zum Einrasten - siehe Fig. 10 - wird der Klemmhalter 1.3 mit zwei Händen auseinandergezogen und der den Dehnbereich 1.3.2 bildende Rohrabschnitt gedehnt. Im gedehnten Zustand - siehe Fig. 11 - wird der Klemmhalter 1.3 auf das Fixierpad 2 aufgesetzt. Bei Rücknahme der Dehnung rasten die Rasthaken 1.3.1 in die korrespondierenden Hinterschnitte 2.1 ein. Zum Ausrasten - siehe Fig. 12 - wird der Klemmhalter 1.3 wieder in gleicher Weise mit zwei Händen auseinandergezogen und im Dehnbereich 1.3.2 gedehnt, sodass sich der Klemmhalter 1.3 kräftefrei vom Fixierpad 2 lösen lässt.

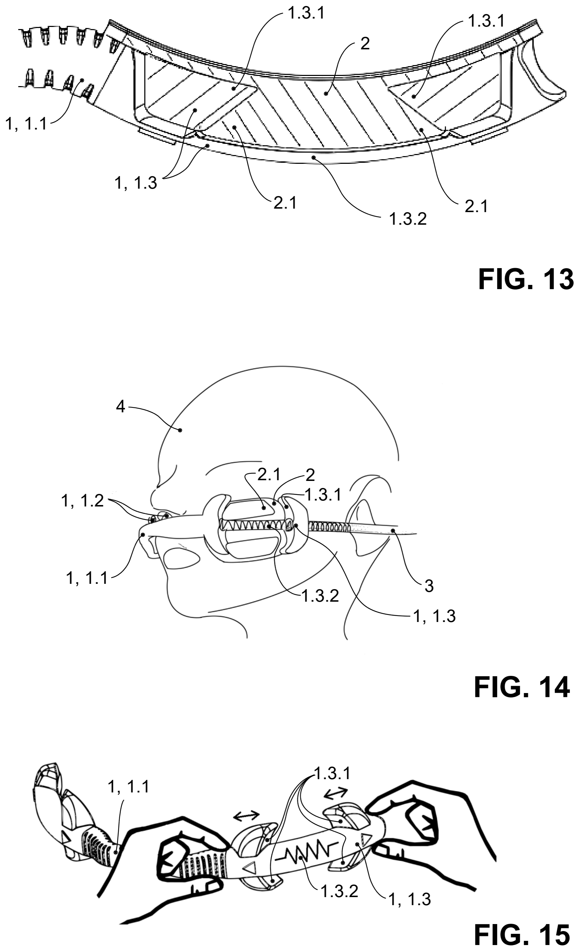

Die symmetrische Gestaltung der Rasthaken 1.3.1 mit ihren aufeinanderzuweisenden Hakenspitzen und die geometrisch korrespondierende Gestaltung der Hinterschnitte 2.1 des Fixierpads 2 veranschaulicht zudem die Schnittdarstellung nach Fig. 13. Die in der Schnittdarstellung erkennbare keilförmige Ausgestaltung der Hakenspitzen der Rasthaken 1.3.1 und die korrespondierende Gestaltung der Hinterschnitte 2.1 unterstützt das Einrasten in die Einrastposition.

Die Fig. 14 und die Fig. 15 zeigen das Halterungssystem gemäß der zweiten Ausführungsform, wobei die Elastizität des den Dehnbereich 1.3.2 bildenden Rohrabschnitts durch eine in den Rohrabschnitt eingearbeitete Spiralfeder realisiert ist. Im Übrigen wird auf die Beschreibung zu den Fig. 5 bis 13 verwiesen.

### Bezugszeichenliste

- 1: Prong
- 1.1: Verteilerstück
- 1.2: Nasenkanüle
- 1.3: Klemmhalter
- 1.3.1: Rasthaken
- 1.3.2: Dehnbereich
- 2: Fixierpads
- 2.1: Hinterschnitt
- 3: Verbindungsschlauch
- 4: Patient

## Patentansprüche

1. Halterungssystem für ein Prong-System zur Beatmungsunterstützung eines Patienten (4), wobei das mittels des Halterungssystems zu halternde Prong-System einen Prong (1) umfasst, der eine oder mehrere Nasenkanülen (1.2) zur Abgabe eines sauerstoffhaltigen Atemgases an den Patienten (4) sowie ein an die Nasenkanülen (1.2) angeschlossenes Verteilerstück (1.1) zur Zuleitung des Atemgases zu den Nasenkanülen (1.2) aufweist, wobei das Halterungssystem ein oder mehrere auf die Wangen des Patienten (4) aufbringbare Fixierpads (2) umfasst, die jeweils eine Klebefläche zur Hautbefestigung aufweisen,
**dadurch gekennzeichnet, dass** das Halterungssystem ein oder mehrere, mit dem Prong (1) fest verbundene oder einen Bestandteil des Prongs (1) bildende Klemmhalter (1.3) umfasst, wobei jeder der Klemmhalter (1.3):
- einem der Fixierpads (2) zugeordnet ist und über eine lösbare Klemmverbindung mit dem zugeordneten Fixierpad (2) verbindbar ist,
- mindestens einen Rasthaken (1.3.1) aufweist, der zur Ausbildung der lösbaren Klemmverbindung in eine an dem Klemmhalter (1.3) zugeordneten Fixierpad (2) ausgeformten, geometrisch mit dem Klemmhalter (1.3) korrespondierenden Hinterschnitt (2.1) normalkraftfrei zur Klebefläche des Fixierpads (2) einrastbar ist,
- einen zum klebeflächen-normalkraftfreien Ein- oder Ausrasten der Klemmverbindung ausgebildeten weich- bis mittelelastischen Dehnbereich (1.3.2), der durch manuell am Klemmhalter (1.3) ausgeübten mechanischen Druck und/oder Zug elastisch verformbar ist.

2. Halterungssystem nach Anspruch 1, **dadurch gekennzeichnet, dass** der weichbis mittelelastische Dehnbereich (1.3.2) des Klemmhalters (1.3) aus einem elastomeren Material aufgebaut ist.

3. Halterungssystem nach Anspruch 1 oder 2, **dadurch gekennzeichnet, dass** das Halterungssystem für jede Wange des Patienten (4) genau eines der Fixierpads (2) und genau einen dem jeweiligen Fixierpad (2) zugeordneten Klemmhalter (1.3) umfasst.

4. Halterungssystem nach einem der Ansprüche 1 bis 3, **dadurch gekennzeichnet, dass** jeder der Klemmhalter (1.3) zwei Rasthaken (1.3.1) aufweist, deren Hakenspitzen spiegelsymmetrisch einander zugewandt sind.

5. Halterungssystem nach Anspruch 4, **dadurch gekennzeichnet, dass** der jeweilige Rasthaken (1.3.1) einen biegeelastischen Schenkel- und Bogenbereich aufweist, an dessen Innenkontur sich der Dehnbereich (1.3.2) erstreckt, wobei der biegeelastische Schenkel- und Bogenbereich des Rasthakens (1.3.1) durch manuell am Klemmhalter (1.3) ausgeübten mechanischen Druck und/oder Zug zum bogenförmigen Ein- oder Ausrasten des Rasthakens (1.3.1) in den oder aus dem ihm zugeordneten Hinterschnitt (2.1) elastisch biegbar ausgebildet ist.

6. Halterungssystem nach Anspruch 5, **dadurch gekennzeichnet, dass** jeder der Klemmhalter (1.3) eine spiegelsymmetrische Form aufweist, wobei die zwei Rasthaken (1.3.1) des Klemmhalters (1.3) spiegelsymmetrisch zur Spiegelsymmetrielinie der spiegelsymmetrischen Form angeordnet sind.

7. Halterungssystem nach Anspruch 4, **dadurch gekennzeichnet, dass** der Klemmhalter (1.3) einen den Dehnbereich (1.3.2) bildenden Rohrabschnitt aufweist, der an das Verteilerstück (1.1) zur Zuleitung des Atemgases angeschlossen ist, wobei an beiden axialen Endbereichen des den Dehnbereich (1.3.2) bildenden Rohrabschnitts jeweils einer der Rasthaken (1.3.1) ausgebildet ist, wobei die beiden an den Endbereichen des Rohrabschnitts ausgebildeten Rasthaken (1.3.1) spiegelsymmetrisch bezüglich des Rohrabschnitts angeordnet sind, und wobei die Hakenspitzen dieser beiden Rasthaken (1.3.1) einander zugewandt sind.

8. Halterungssystem nach Anspruch 7, **dadurch gekennzeichnet, dass** die Rasthaken (1.3.1) in Ankerform ausgebildet sind.

9. Prong-System zur Beatmungsunterstützung eines Patienten (4), umfassend:
- einen Prong (1), der ein oder mehrere Nasenkanülen (1.2) zur Abgabe eines sauerstoffhaltigen Gases an den Patienten (4) sowie ein an die Nasenkanüle (1.2) angeschlossenes Verteilerstück (1.1) zur Zuleitung des sauerstoffhaltigen Atemgases zu den Nasenkanülen (1.2) aufweist,
- ein Halterungssystem zur Halterung des Prongs (1) an den Wangen des Patienten (4), wobei das Halterungssystem ein oder mehrere auf die Wangen des Patienten (4) aufbringbare Fixierpads (2) umfasst, die jeweils eine Klebefläche zur Hautbefestigung aufweisen,
- einen Verbindungsschlauch (3) zur Zuleitung des Atemgases zum Prong (1),
**dadurch gekennzeichnet, dass** das Halterungssystem ein Halterungssystem nach einem der Ansprüche 1 bis 8 ist.

10. Prong-System nach Anspruch 9, **dadurch gekennzeichnet, dass** der oder die Klemmhalter (1.3) einen integralen Bestandteil des Prongs (1) bilden, wobei der die Nasenkanülen (1.2), das Verteilerstück (1.1) und den Klemmhalter (1.3) umfassende Prong (1) als monolithisches Bauteil ausgebildet ist.
